# EUROPEAN PATENT APPLICATION

(11) **EP 3 388 072 A1**
(43) Date of publication of application: **17.10.2018**
(21) Application number: 17165728.1
(22) Date of filing: 10.04.2017
(51) Int. Cl.: A61K 38/43, A61P 3/10, A61P 9/00, A61P 19/02, A61P 25/28, C12N 5/10, C12N 15/00

(54) **UBIQUINONE-INDEPENDENT CYTOPLASMIC DIHYDROOROTATE DEHYDROGENASE FOR USE AS MEDICAMENT**

(71) Applicant: Universität Leipzig, 04109 Leipzig (DE)
(72) Inventor: SEIBEL, Peter, 97292 Uettingen (DE)
(74) Representative: Schüssler, Andrea

(57) **Abstract**

The present invention relates to a ubiquinone-independent cytoplasmic DHODH (DHODH), and/or to a nucleic acid molecule comprising a nucleotide sequence encoding the ubiquinone-independent DHODH, and/or to a recombinant expression vector comprising the nucleic acid molecule comprising the nucleotide sequence encoding the ubiquinone-independent DHODH for use as medicament or rather for use in the treatment of mitochondrial diseases. Such mitochondrial diseases are associated with a defective functional link between the pyrimidine *de novo* biosynthesis and the mitochondrial OXPHOS and are accompanied with pyrimidine auxotrophy. According to the present invention, such pyrimidine auxotrophy is rescued by xenoexpression of a simple single-protein enzyme, i.e. of a DHODH from other eukaryotic species, which is ubiquinone-independent so that the pyrimidine de *novo* biosynthesis is not linked to the mitochondrial OXPHOS.

## Description

### Field of the Invention

The present relates to a ubiquinone-independent cytoplasmic dihydroorotate dehydrogenase (DHODH) and/or to a nucleic acid molecule comprising a nucleotide sequence encoding the ubiquinone-independent DHODH, e.g. *URA1* gene, and/or to the recombinant expression vector comprising the nucleic acid molecule comprising a nucleotide sequence encoding the ubiquinone-independent DHODH for use as medicament. The present invention further relates to a transgenic non-human animal.

### Background of the Invention

Mitochondria are the powerhouses of eukaryotic cells. In these organelles, the production of ATP is coupled to the oxygen consumption by the oxidative phosphorylation system (OXPHOS). The 13 protein subunits of the OXPHOS complexes are encoded by the mitochondrial DNA (mtDNA), wherein approximately 1,500 additional proteins are enciphered by the nuclear genome and involved in the organelle biogenesis (Taylor, S. W. et al., Trends Biotechnol 21, 82-8 (2003); Taylor, S. W. et al. Nat Biotechnol 21, 281-6 (2003)). Thus, aside from their bioenergetic function, mitochondria are involved in a large number of other cellular functions. These include the reactive oxygen/nitrogen species (ROS/RNS) metabolism (Brown, G. C., Front Biosci 12, 1024-33 (2007); Murphy, M. P., Biochem J 417, 1-13 (2009)), apoptosis, calcium (Rimessi, A. et al., Biochim Biophys Acta 1777, 808-16 (2008)) or iron (Dunn, L. L. et al., Trends Cell Biol 17, 93-100 (2007)) homeostasis, and intracellular signal transduction as well as the specific compartmentalization of a variety of metabolic pathways (energy production, urea cycle, nucleotide and heme metabolism, lipids and aminoacids metabolism, signal transduction), wherein an increasing number of proteins with unknown or unproved function(s) are also predicted to have a mitochondrial localization.

Such well-documented involvement of this organelle in cellular functions other than energy production makes the mitochondria the best candidate to represent the main cellular metabolic branching and integration point. Even in the specific case of the mitochondrial respiratory complexes, some proteins have been shown to play unexpected roles, as in the case of the apoptotic function of cytochrome c (Hüttemann, M. et al. Mitochondrion 11, 369-81 (2011); Liu, X. et al., Cell 86, 147-57 (1996)) or the tumor suppressor activity of succinate dehydrogenase (Bardella, C. et al., Biochim Biophys Acta 1807, 1432-43 (2011). Following, a fundamental biochemical crosslink between mitochondria and nucleus is often underestimated. For example, the pyrimidine *de novo* biosynthesis links basic cellular needs to mitochondria and, thus, plays a pivotal role.

To serve cells need, the pyrimidine pool can be filled generally via three independent pathways, i.e. via the pyrimidine *de novo* biosynthesis from metabolites as mentioned above, the endogenous salvage pathway, in which purines and pyrimidines are synthesized from intermediates in the degradative pathway for nucleotides, and the uptake of preformed nucleosides and bases from the extracellular environment through active and passive transporters (Cass, C. E. et al., Biochem Cell Biol 76, 761-70 (1998)). While in fully differentiated or resting cells, the pyrimidine demand is widely covered by the salvage pathway (Fairbanks, I. D. et al., J Biol Chem 270, 29682-689 (1995)), the pyrimidine *de novo* biosynthesis is indispensable in proliferating cells because pyrimidines are required to serve the great demand of nucleic acid building blocks during mitosis, i.e. cell proliferation. Thus, sustaining proliferative cells metabolism largely depends on the functionality of the enzymes catalyzing the pathway of pyrimidine *de novo* biosynthesis.

Such pathway consists of a number of enzymes that includes the dihydroorotate dehydrogenase (DHODH) as an essential keyplayer. The DHODH gene encodes dihydroorotate dehydrogenase. A cDNA sequence for human DHODH is disclosed in GenBank NM_001361 (https://www.ncbi.nlm.nih.gov/nuccore/NM 001361). DHODH is an enzyme that catalyzes the fourth step in the *de novo* biosynthesis of pyrimidines, which converts dihydroorotate to orotate. In vertebrates, the DHODH is located on the outer face of the inner mitochondrial membrane and belongs to the membrane bound class 2 dihydroorotate dehydrogenases that uses ubiquinone (Q) as electron acceptor. It functionally links the pyrimidine *de novo* biosynthesis to the mitochondrial respiratory chain, i.e. to the mitochondrial OXPHOS, thus, ensuring pyrimidine prototrophy in vertebrates.

As mitochondria are taking a center stage in many aspects of cellular pathophysiology, apart from their classical and fundamental role in ATP production via OXPHOS, it follows that the impairment of the organelle's function and/or biogenesis leads to a variety of tissue/cell-specific defects that range from defects in e.g. the energy metabolism or in the pyrimidine *de novo* biosynthesis to complex diseases with a mitochondrial association, including Alzheimer's, Parkinson's, and Huntington's disease, Miller syndrome, also known as Genee-Wiedemann syndrome, Wildervanck-Smith syndrome or postaxial acrofacial dystosis (POADS), cancer, type 2 diabetes, cardiovascular disease, and osteoarthritis (Taylor, S. W. et al. Nat Biotechnol 21, 281-6 (2003)). However, it has been previously shown that xenoexpression of relatively simple single-protein enzymes from different organisms in human cells can be adopted to treat mitochondrial diseases caused by OXPHOS defects or mitochondrial dysfunctions. For example, mitochondrial complex I and complex IV defects have been rescued by xenoexpression of the *Saccharomyces cerevisae* NDI1 gene encoding a rotenone-insensitive NADH:ubiquinone oxidoreductase (Seo, B. B. et al., Proc Natl Acad Sci USA 95, 9167-71(1998)) and the *Ciona intestinalis* AOX gene encoding a cyanide-resistant terminal oxidase (Dassa, E. P. et al., EMBO Mol Med 1, 30-6 (2009)). Furthermore, transgenic mice expressing the AOX gene have recently been generated that can be used as unique tool to study the possibility to treat the mitochondrial diseases associated with OXPHOS defects (El-Khoury, R. et al., PLoS Genet 9, e1003182 (2013)). Moreover, uridine, or the prodrug triacetyluridine (PN401) supplementation has been studied for the treatment of different pathological states directly caused by OXPHOS defects (Garcia, R. A. et al., Brain Res 1066, 164-71 (2005); López-Martin, J. M. et al., Hum Mol Genet 16, 1091-97 (2007)) and for the treatment of diseases that are associated with mitochondrial dysfunctions, such as Alzheimer's (Saydoff, J. A. et al., J Alzheimers Dis 36, 637-57 (2013)), Parkinson's (Klivenyi, P. et al., Neuromolecular Med 6, 87-92 (2004)), Huntington disease (Saydoff, J. A. et al., Brain Res 994, 44-54 (2003)) and Miller syndrome (Wiedemann, H.-R., Klin Päd 185, 181-86 (1973)).

However, the prior art does not describe any tool that can be used to treat mitochondrial disorders or malfunctions which are associated with a defective functional link between the pyrimidine *de novo* biosynthesis and the mitochondrial OXPHOS. Thus, there is a need for a molecule (e.g. polypeptide or nucleic acid) that can be used to treat such mitochondrial diseases or malfunctions.

### Object of the present Invention

The object of the invention is to provide a tool (e.g. polypeptide or nucleic acid) that can be used to treat mitochondrial diseases, which are associated with a defective functional link between the pyrimidine *de novo* biosynthesis and the mitochondrial OXPHOS.

### Brief Description of the Invention

This object is solved by the subject-matter of present independent claim 1. Preferred embodiments are mentioned in the dependent claims.

In vertebrates, the pyrimidine *de novo* biosynthesis pathway is functionally linked to mitochondria because one of the enzymes of the pyrimidine *de novo* biosynthesis pathway, the dihydroorotate dehydrogenase (DHODH), is located on the outer face of the inner mitochondrial membrane.

Since the DHODH belongs to the membrane bound class 2 DHODHs that use ubiquinone (Q) as electron acceptor, it is functionally linked to the respiratory chain and, thus, to the mitochondrial oxidative phosphorylation system (OXPHOS). Following, mitochondrial diseases, which are associated with a defective functional link between the pyrimidine *de novo* biosynthesis and the mitochondrial OXPHOS, are characterized by pyrimidine auxotrophy.

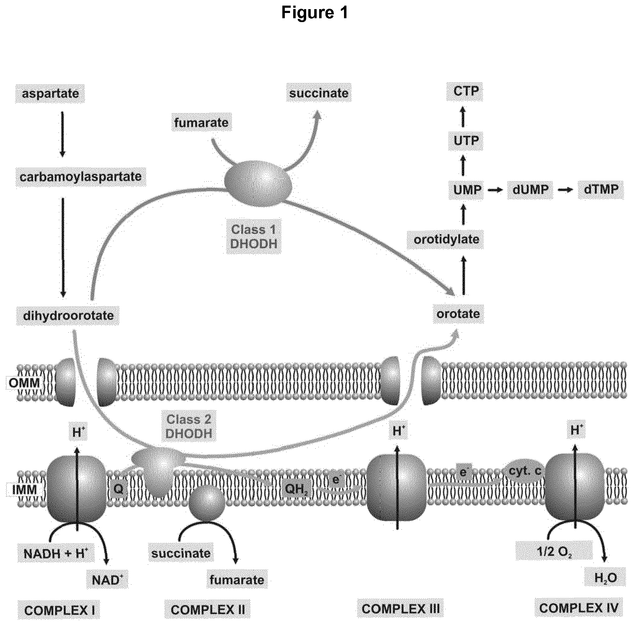

Such pyrimidine auxotrophy, however, can be rescued by xenoexpression of a cytoplasmic DHODH from other eukaryotic species, which is ubiquinone-independent so that the pyrimidine *de novo* biosynthesis is not linked to the mitochondrial OXPHOS. For example the eukaryotic species yeast, e.g. S. *cerevisiae,* adapt soluble class 1 enzymes located in the cytoplasm, wherein these enzymes utilize fumarate as electron acceptor (Class 1 DHODH, EC 1.3.98.1, Fig. 1). Two other Class 1 DHODHs, which use NAD+ or NADP+, are EC 1.3.1.14 (NAD+) in bacteria (e.g. Bacillus subtilis) and EC 1.3.1.15 (NADP+)

### [https://www.ncbi.nlm.nih.gov/pubmed/4380263]

Thus, the present invention relates to a ubiquinone-independent DHODH, and/or to a nucleic acid molecule comprising a nucleotide sequence encoding the ubiquinone-independent yDHODH, and/or to a recombinant expression vector comprising the nucleic acid molecule comprising the nucleotide sequence encoding the ubiquinone-independent yDHODH for use as medicament or rather for use in the treatment of mitochondrial diseases or malfunctions, which are associated with a defective functional link between the pyrimidine *de novo* biosynthesis and the mitochondrial OXPHOS, are characterized by pyrimidine auxotrophy.

### Brief description of the Figures:

**Figure 1****: *Pyrimidine* de novo *biosynthesis in vertebrates and in yeasts.***
   Figure 1 represents the schematic representation of the pyrimidine *de novo* biosynthesis in vertebrates and in other eukaryotic species such as yeasts, e.g. S. *cerevisiae.* In vertebrates, the DHODH of the pyrimidine biosynthesis pathway is located on the outer face of the inner mitochondrial membrane and belongs to the membrane bound class 2 dihydroorotate dehydrogenases (Class 2 DHODH, EC 1.3.5.2) that uses ubiquinone as electron acceptor. Other eukaryotic species, such as yeasts, e.g. *S*. *cerevisiae,* adapt soluble class 1 enzymes located in the cytoplasm, wherein these enzymes utilize fumarate as electron acceptor (Class 1 DHODH, EC 1.3.98.1), so that pyrimidine *de novo* biosynthesis is not linked to the mitochondrial OXPHOS.

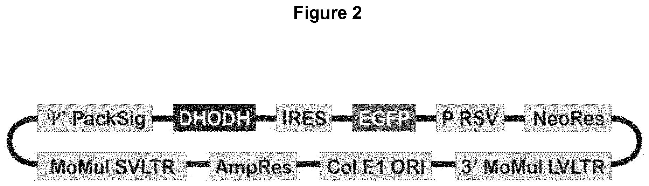

**Figure 2****: *Vector introducing* S. cerevisiae *DHODH into human cells.***
   Figure 2 represents a recombinant expression vector introducing *S*. *cerevisiae* yDHODH into human cells, more specifically, a retroviral *URA1* gene expression system. Besides other constructive elements, the vector contains the information of the yeast isoform of DHODH (equivalent to URA1) and EGFP, separated by an internal ribosomal entry site. Thus, proteins are translated independently from the same message.
**Figure 3****: *Growth curve of 143b.TK⁻ and 143b.TK⁻.Rho⁰ cells supplemented with yDHODH from* S. cerevisiae.**
   Figures 3a, 3b, 3c and 3d represent the growth curves of cells of the proliferative human osteosarcoma cell line 143b.TK- displaying a pyrimidine prototrophy and of its derivative Rho⁰ (ρ⁰) cell line 143b.TK⁻. Rho⁰, a cell line that is devoid of endogenous mitochondrial genomes and, thus, exhibiting a uridine auxotrophy, with (Fig. 3c, 143b.TK⁻.DHODH; Fig. 3d, 143b.TK⁻.Rho⁰.DHODH) and without (Fig. 3a, 143b.TK⁻; Fig. 3b, 143b.TK⁻.Rho⁰) supplementation with yDHODH from *S*. *cerevisiae* and with (blue color: uridine +) and without (red color: uridine -) supplementation with uridine.]
**Figure 4****: *FACS analysis of 143b.TK⁻.Rho⁰ and 143b.TK⁻.Rho⁰.DHODH.***
   Figures 4a and 4b display the results of a FACS analysis derived from a time trace (0 h, 3 h, 6 h, 9 h, 12 h, 24 h, 5 d) of cultured 143b.TK⁻.Rho⁰ cells and 143b.TK⁻.Rho⁰.DHODH cells in the absence of uridine.
**Figure 5****: *Releasing 143b.Rho⁰ cells from cell cycle arrest by uridine complementation.***

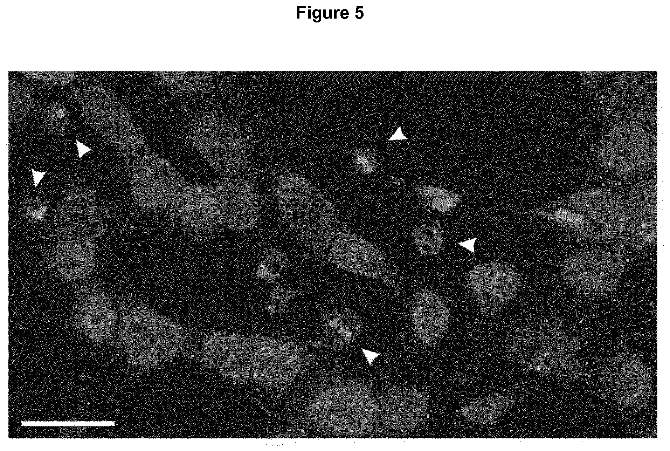

   Figure 5 displays a confocal image of 143b.TK⁻.Rho⁰ cells, which have been cultured for approximately 12 hours without uridine supplementation, 10 hours post uridine supplementation. The white arrowheads indicate cells in metaphase/anaphase transition (red color: mito-dsRED illuminating mitochondrial network; green color: pico-green staining of the nuclear DNA). The white bar reflects 20 µm.

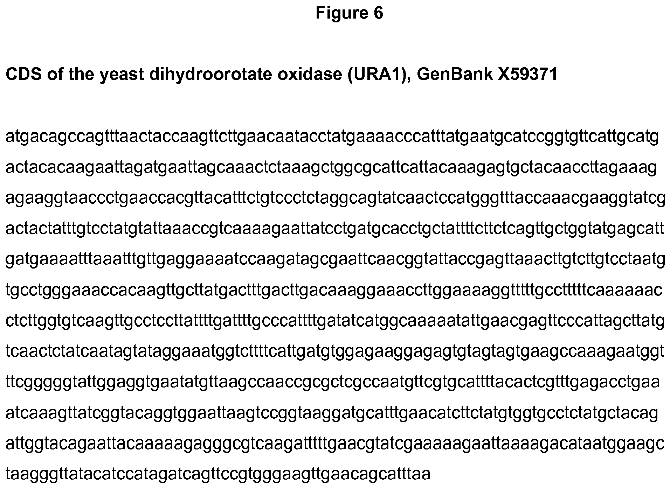

**Figure 6****: *CDS* of *the yeast dihydroorotate oxidase (URA1), GenBank X59371***

### Detailed Description of the Invention

The present invention relates to a cytoplasmic dihydroorotate dehydrogenase (DHODH), and/or to the nucleic acid molecule comprising a nucleotide sequence encoding the DHODH, and/or to the recombinant expression vector comprising the nucleic acid molecule comprising the nucleotide sequence encoding the DHODH for use as a medicament. The present invention further relates to a transgenic non-human animal which can be used as unique tool to study candidate substances or new treatment approaches for mitochondrial malfunctions based on pyrimidine auxtrotrophy.

Dihydroorotate dehydrogenase (DHODH; PyrE) is involved in the *de novo* synthesis of pyrimidines, catalyzing the oxidation of dihydroorotate to orotate. Two classes of DHODH have been described on the basis of differences in amino acid sequence; Class II DHODH are found in most fungi (including *A. fumigates and C. albicans),* animals, plants, gram-negative bacteria and archeabacteria. These use an FMN molecule as a cofactor, and, in the case of humans and fungi, this is recycled by means of oxidation via a quinine cofactor from the respiratory chain. The human and fungal proteins are non-covalently associated with the mitochondrial inner membrane by an N-terminal trans-membrane domain. The quinine-binding pocket is adjacent to, but distinct from the catalytic site of the enzyme. Class I enzymes are found in gram-positive bacteria, trypanosomes, *Saccharomyces cerevisiae,* and closely related fungi such as other members of the genus *Saccharomyces.*

It is to be understood that the term "isolated from" may be read as "of' herein. Therefore, references to proteins being "isolated from" a particular organism include proteins which were prepared by means other than obtaining them from the organism, such as synthetically or recombinantly.

Unless otherwise indicated, the recombinant DNA techniques utilized in the present invention are standard procedures, well known to those skilled in the art. Such techniques are described and explained throughout the literature in sources such as, J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), DNA Cloning: A Practical Approach, Volumes 1-4, IRL Press (1995 and 1996), and F.M. Ausubel et al (Editors), Current Protocols in Molecular Biology, Greene Pub. Associates and Wiley-Interscience (1988, including all updates until present). In particular, these documents describe in detail methods of transcribing or replicating nucleic acid molecules and suitable conditions required therefor.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The term "biologically active" or "functional" refers to a polypeptide that exhibits at least one of the functional characteristics attributed to native DHODH.

As used herein, the term "gene" refers to a polynucleotide containing at least one open reading frame that is capable of encoding a particular polypeptide or protein after being transcribed or translated. The term "(gene) construct" refers to a nucleic acid molecule, e.g., a vector, containing the subject polynucleotide, operably linked in a manner capable of expressing the polynucleotide in a host cell. As used herein, the term "polynucleotide" encompasses deoxyribonucleic acid (DNA), and, where appropriate, ribonucleic acid (RNA). As used herein the term also encompasses analogs of either RNA or DNA made from nucleotide analogs, and, as applicable to the embodiment being described, single-stranded (such as sense or antisense) and double-stranded polynucleotides.

As used herein, the terms "coding sequence" or "a sequence with encodes a particular protein", denotes a nucleic acid sequence which is transcribed (in the case of DNA) and translated (in the case of mRNA) into a polypeptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and even synthetic DNA sequences.

By "operably linked" is meant that a DNA sequence and a regulatory sequence are connected in such a way as to permit gene expression when the appropriate molecules (e.g., transcriptional activator proteins) are bound to the regulatory sequence.

By "operatively inserted" is meant that a nucleotide sequence of interest is positioned adjacent a nucleotide sequence that directs transcription and translation of the introduced nucleotide sequence of interest.

The term "corresponds to" or "corresponding to" is meant homologous to or substantially equivalent to or functionally equivalent to the designated sequence.

The term "dihydroorotate dehydrogenase" or "DHODH" as used in the present invention refers to an enzyme that catalyzes the fourth step in the pyrimidine *de novo* biosynthesis. It converts dihydroorotate to orotate. In vertebrates, the DHODH is located on the outer face of the inner mitochondrial membrane and belongs to the membrane bound class 2 dihydroorotate dehydrogenases (E.C. 1.3.5.2) that uses ubiquinone (Q) as the proximal and cytochrome c oxidase as the ultimate electron acceptor (Fig. 1). The other enzymes of the pyrimidine *de novo* biosynthesis are present as multifunctional proteins in the cytosol which is the compartment where biosynthetic pathways usually take place. Therefore, the functional link of the pyrimidine *de novo* biosynthesis to the mitochondrial respiratory chain, i.e. to the mitochondrial oxidative phoshorylation system (OXPHOS) is the result of an extraordinary compartmentation preserved in eukaryotic cells.

The term "mitochondrial oxidative phosphorylation system" or "OXPHOS" as used in the present invention is the metabolic pathway in which the mitochondria in cells use their structure, enzymes and energy released by the oxidation of nutrients to reform ATP, which is the molecule that supplies energy to metabolism. During oxidative phosphorylation, electrons are transferred from electron donors to electron acceptors, such as oxygen, in redox reactions, wherein these redox reactions release energy, which is used to form ATP. More specifically, in the inner mitochondrial membrane, electrons from NADH and succinate pass through the electron transport chain (ETC), also known as respiratory chain, to oxygen which is reduced to water. The transfer of electrons through ETC results in the pumping of H⁺ across the membrane creating a proton gradient across the membrane, which is used by the ATP synthase (located on the membrane) to generate ATP, wherein ubiquinone functions as an electron carrier from enzyme complex I and enzyme complex II to complex III in this process. Thus, ubiquinone functions in every cell of the vertebrates on the one hand to synthesize energy and on the other hand to synthesize pyrimidines, since the DHODH also uses ubiquinone as electron acceptor.

The "pyrimidine" and "purine" (deoxy)nucleotides are the building blocks of DNA and RNA, wherein three nucleobases found in nucleic acids are pyrimidine derivatives, cytosine (C), thymine (T), and uracil (U), and two nucleobases found in nucleic acids are purines, adenine (A) and guanine (G). "Uridine" is one of the four basic components of RNA, the other three are "adenosine", "guanosine", and "cytidine", wherein uridine refers to a nucleoside that contains an uracil attached to a ribose (known as ribofuranose) via a β-N₁-glycosidic bond. Furthermore, uridine is known as "deoxyuridine" if uracil is attached to a deoxyribose ring.

To serve cells' needs, the pyrimidine pool can be filled generally via three independent pathways, i.e. via the pyrimidine *de novo* biosynthesis from metabolites, the endogenous salvage pathway, in which purines and pyrimidines are synthesized from intermediates in the degradative pathway for nucleotides, and the uptake of preformed nucleosides and bases from the extracellular environment through active and passive transporters. While in fully differentiated or resting cells, the pyrimidine demand is widely covered by the salvage pathway, the pyrimidine *de novo* biosynthesis is indispensable in proliferating cells because pyrimidines are required to serve the great demand of nucleic acid building blocks during mitosis, i.e. cell proliferation. Thus, sustaining proliferative cells metabolism largely depends on the functionality of the enzymes catalyzing the pathway of the pyrimidine *de novo* biosynthesis in order to ensure pyrimidine prototrophy. Following, a defective functional link between the pyrimidine *de novo* biosynthesis and the mitochondrial OXPHOS leads to a variety of tissue/cell-specific defects, i.e. to a variety of mitochondrial diseases.

Such mitochondrial diseases or malfunctions of the present invention which are associated with a defective functional link between the pyrimidine *de novo* biosynthesis and the mitochondrial OXPHOS, are caused by alterations or rather mutations in the genes, encoding the enzymes, which catalyze the steps of the pyrimidine *de novo* biosynthesis and/or in the genes encoding the enzymes, which catalyze the mitochondrial OXPHOS and/or in the genes encoding the respiratory chain subunits and assembly genes that regulate their transport to the mitochondria. Such alterations or rather mutations result in a defective or inoperative DHODH catalyzing the fourth step in the pyrimidine *de novo* biosynthesis so that mitochondrial diseases, which are associated with a defective functional link between the pyrimidine *de novo* biosynthesis and the mitochondrial OXPHOS are accompanied with pyrimidine auxotrophy.

Sometimes the mitochondrial malfunctions are not due to a permanent alteration or mutation of a gene encoding an enzyme which catalyzes the steps of the pyrimidine *de novo* biosynthesis but are initiated as a side effect of other medicaments. In this regard it has been observed by the inventors that many antibiotics stop the activity of the DHODH and lead to pyrimidine auxotrophy. The human DHODH catalyzes the fourth step in the pyrimidine de novo synthesis, thereby utilizing ubiquinone as electron acceptor of the overall reaction. It is important to understand that many antibiotics that act on protein biosynthesis in bacteria can display a severe side effect in human mitochondria as their ribosomes are evolutionary highly related to the bacterial ribosome and thus sensible to antibiotics. As a consequence, a block of mitochondrial protein biosynthesis upon antibiotic therapy will induce a severe deficiency of the respiratory chain that in turn can no longer function as ubiquinone electron acceptor. Thus, the pyrimidine biosynthesis via DHODH that under normal conditions pumps the electrons via ubiquinone into the respiratory chain is stalled.

According to the present invention, such pyrimidine auxotrophy is rescued by xenoexpression of a simple single-protein enzyme, i.e. of a cytoplasmic DHODH from other eukaryotic species, which is ubiquinone-independent so that the pyrimidine de *novo* biosynthesis is not linked to the mitochondrial OXPHOS. Thus, in one embodiment of the present invention, the term "DHODH" or "ubiquinone-independent DHODH" refers to a DHODH originating from other eukaryotic species, such as yeast (yDHODH), e.g. S. *cerevisiae.* The yeast S. *cerevisae* adapts soluble class 1 enzymes located in the cytoplasm, wherein these enzymes utilize fumarate as electron acceptor (Fig. 1). In a specific embodiment of the present invention, the ubiquinone-independent yDHODH is the Class 1 DHODH, EC 1.3.98.1. In a specific embodiment the DHODH is wildtype or a variant thereof. The variants include, for instance, naturally-occurring variants due to allelic variations between individuals (e.g., polymorphisms), alternative splicing forms, etc. Variants preferably exhibit a nucleotide sequence identify of typically at least about 75% preferably at least about 85%, more preferably at least about 90%, more preferably at least about 95%. Variants of a DHODH gene also include nucleic acid sequences, which hybridize to a sequence as defined above (or a complementary strand thereof) under stringent hybridization conditions. Typical stringent hybridization conditions include temperatures above 30°C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

In another embodiment, the present invention is directed to a nucleic acid molecule comprising a nucleotide sequence encoding the DHODH of the present invention. The URA1 gene has the GenBank accession No. X59371 (https://www.ncbi.nlm.nih.aov/nuccore/X59371). The coding sequence is shown in Fig. 6.

Furthermore, the present invention is directed to a recombinant expression vector comprising the nucleic acid molecule comprising the nucleotide sequence encoding DHODH (e.g. Fig. 2). Preferably, the recombinant expression vector is constructed on the basis of a plasmid, cosmid, virus, bacteriophage or represents another vector usually used in the field of genetic engineering. More preferably, the recombinant expression vector of the present invention is constructed on the basis of a virus, e.g. adenovirus, herpes virus, vaccinia, or, more preferably, of a RNA virus such as a retrovirus.

In a preferred embodiment, the vector use according to the invention is a non-viral vector. Typically, the non-viral vector may be a plasmid vector, e.g. pBApo-CMV Neo, pIRES2-EGFP or pLXRN (all Clontech Laboratories, Inc., Mountain View, CA, USA).

The terms "Gene transfer" or "gene delivery" refer to methods or systems for reliably inserting foreign DNA into host cells. Such methods can result in transient expression of non integrated transferred DNA, extrachromosomal replication and expression of transferred replicons (e.g., episomes), or integration of transferred genetic material into the genomic DNA of host cells.

In a more preferred embodiment the vector is a viral vector. Gene delivery viral vectors useful in the practice of the present invention can be constructed utilizing methodologies well known in the art of molecular biology. Typically, viral vectors carrying heterologous genes are assembled from polynucleotides encoding the heterologous gene (DHODH), suitable regulatory and/or control elements and elements necessary for production of viral proteins which mediate cell transduction. Examples of viral vector include adenoviral, retroviral, lentiviral, herpesvirus and adeno-associated virus (AAV) vectors.

Such recombinant viruses may be produced by techniques known in the art, such as by transfecting packaging cells or by transient transfection with helper plasmids or viruses. Typical examples of virus packaging cells include PA317 cells, PsiCRIPO cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

Even more preferably, the viral vector is a retrovirus, e.g. a derivative of a murine or avian retrovirus. In a preferred embodiment, the retrovirus is selected from the group consisting of a Moloney murine leukemia virus (MoMul or MoMuLV), Harvey murine sarcoma virus (HaMuSV), murine mammary tumor virus (MuMTV), Rous sarcoma virus (RSV), and gibbon ape leukemia virus (GaLV)

The control elements are selected to be functional in a mammalian cell.

Alternatively, heterologous control sequence can be employed. Useful heterologous control sequences generally include those derived from sequences encoding mammalian or viral genes. Examples include, but are not limited to, the phophoglycerate kinase (PKG) promoter, CAG, neuronal promoter, mouse mammary tumor virus LTR promoter; adenovirus major late promoter (Ad MLP); a herpes simplex virus (HSV) promoter, a cytomegalovirus (CMV) promoter such as the CMV immediate early promoter region (CMVIE), rous sarcoma virus (RSV) promoter, synthetic promoters, hybrid promoters, and the like. In addition, sequences derived from nonviral genes, such as the murine metallothionein gene, will also find use herein. Such promoter sequences are commercially available from, e.g. Stratagene (San Diego, CA). For purposes of the present invention, both heterologous promoters and other control elements, such as CNS-specified and inducible promoters, enhancers and the like, will be of particular use.

Examples of heterologous promoters include the CMV promoter. Examples of CNS specific promoters include those isolated from the genes from myelin basis protein (MBP), glial fibrillary acid protein (GFAP), and neuron specific enolase (NSE).

Examples of inducible promoters include DNA responsive elements for ecdysone, tetracycline, hypoxia andaufin.

Furthermore, the recombinant expression vector may comprise a polyadenylation signal that is used to ensure the polyadenylation of the mRNA and to participate to correct termination of transcription. Preferably, the polyadenylation signal can be selected from the group consisting of SV40 poly A (Simian virus 40 poly A), HSV TK poly A (herpes simplex virus (HSV) type 1 thymidine kinase), hGH poly A (human growth hormone), hGH terminator (human growth hormone) or BGH poly A (bovine growth hormone). Moreover, the recombinant expression vector of the present invention comprises preferably a nucleotide sequence that allows for translation initiation, e.g. an internal ribosome entry site (IRES).

In another embodiment of the present invention, the recombinant expression vector may comprise a long terminal repeat that is used to enhance the transformation, such as a retrovirus long terminal repeat, e.g. of the Simian virus (SVLTR), lentivirus (LVLTR), MMLV LTR (Moloney murine sarcoma virus) or MSCV LTR (Murine Stem Cell Virus). Furthermore, the recombinant expression vector may comprise a reporter gene that is used as an indication of whether the nucleic acid molecule has been taken up by or expressed in the vertebrate cell or subject to be treated. In one embodiment of the present invention, the reporter gene can be selected from the group consisting of the genes *lacZ* (encoding the β-galactosidase), *phoA* (encoding the alkaline phosphatise, AP), *cat* (encoding the chloramphenicol acetyltransferase, CAT), *gus*B (encoding the β-glucuronidase, GUSB), *luc* (encoding the luciferase from firefly *Photinus pyralis* and *Renilla reniformis*), *gfp* (encoding the green fluorescent protein, GFP), *egfp* (encoding the enhanced green fluorescent protein, EGFP), *bfp* (encoding the blue fluorescent protein, BFP), *cfp* (encoding the cyan fluorescent protein, CFP), and *ecfp* (encoding the enhanced fluorescent protein, ECFP). In another embodiment of the present invention, the reporter gene can be an antibiotic-resistance gene that can be selected from the group consisting of an ampicillin-(AmpRes), neomycin- (NeoRes), kanamycin- (KanRes), tetracycline- (TetRes), streptomycin- (StreptRes) or chloramphenicol-resistance gene. In a preferred embodiment, the recombinant expression vector comprises more than one reporter genes, e.g. two reporter genes, such as EGFP or ECFP with NeoRes. Other suitable reporter/marker genes are preferably selected from one or more elements of the group consisting of Herpes Simplex Virus thymidine kinase gene, geneticin gene, zeocin gene, puromycin gene, cytosine deaminase gene, hygromycin B gene, secreted alkaline phosphatase gene, guanine phosphoribosyl transferase (gpt) gene, alcohol dehydrogenase gene, hypoxanthine phosphoribosyl transferase (HPRT) gene, cytochrome P450 gene and/or toxin genes such as α subunit of diphtheria, pertussis toxin, tetanus toxoid.

In addition, the recombinant expression vector may comprise a prokaryotic or eukaryotic replication origin.

It is herein provided a method for treating a mitochondrial dysfunction based on pyrimidine auxotrophy in a subject, said method comprising:
(a) providing a vector as defined above, which comprises a DHODH encoding nucleic acid; and
(b) delivering the vector to the subject, whereby said vector transduces cells in the subject's cells, and whereby DHODH is expressed by the transduced cells at a therapeutically effective level.

The vectors used herein may be formulated in any suitable vehicle for delivery. For instance they may be placed into a pharmaceutically acceptable suspension, solution or emulsion. Suitable mediums include saline and liposomal preparations. More specifically, pharmaceutically acceptable carries may include sterile aqueous solutions, suspensions, and emulsions. Examples of non-aqueous solvents are propylene glycol, polyethylene glycol, vegetable oils such as olive oil, and injectable organic esters such as ethyl oleate. Aqueous carries include water, alcoholic/aqueous solutions, emulsions or suspensions, including saline and buffered media. Intravenous vehicles include fluid and nutrient replenishers, electrolyte replenishers (such as those based on Ringer's dextrose), and the like.

Preservatives and other additives may also be present such as, for example, antimicrobials, antioxidants, chelating agents, and insert gases and the like.

A colloidal dispersion system may also used for targeted gene delivery. Colloidal dispersion system include macromolecule complexes, nanocapsules, microspheres, beads, and lipid-based systems including oil-in-water emulsions, micelles, mixed micelles, and liposomes.

The preferred doses and regimen may be determined by a physician, and depend on the age, sex, weight, of the subject, and the stage of the disease. As an example, for delivery of cholesterol 24-hydroxaxylase using a viral expression vector, each nit dosage of cholesterol 24-hydroxylase expressing vector may comprise 2.5 to 100 µl of a composition including a viral expression vector in a pharmaceutically acceptable fluid at a concentration ranging from 10¹¹ to 10¹⁶ viral genome per ml for example.

A further object of the invention concerns a pharmaceutical composition which comprises a therapeutically effective amount of a vector according to the invention.

By a "therapeutically effective amount" is meant a sufficient amount of the vector of the invention to treat the mitochondrial malfunction at a reasonable benefit/risk ratio applicable to any medical treatment.

It will be understood that the total daily dosage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specified compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range per adult per day. The therapeutically effective amount of the vector according to the invention that should be administered, as well as the dosage for the treatment of a pathological condition with the number of viral or non-viral particles and/or pharmaceutical compositions of the invention, will depend on numerous factors, including the age and condition of the patient, the severity of the disturbance or disorder, the method and frequency of administration and the particular peptide to be used.

The presentation of the pharmaceutical compositions that contain the vector according to the invention may be in any form that is suitable for intracerebral, intrathecal, intraventricular or intravenous administration. In the pharmaceutical compositions of the present invention for intramuscular, intravenous, intracerebral, intrathecal or intraventicular administration, the active principle, alone or in combination with another active principle, can be administered in a unit administration form, as a mixture with conventional pharmaceutical supports, to animals and human beings.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The vector according to the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine, and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

Multiple doses can also be administered.

In a further embodiment, the present invention relates to a recombinant host cell transiently or stable containing the nucleic acid molecule comprising the nucleotide sequence encoding the ubiquinone-independent yDHODH of the present invention or the recombinant expression vector comprising the nucleic acid molecule comprising the nucleotide sequence encoding the ubiquinone-independent yDHODH of the present invention. A "host cell" is understood to a be an organism that is capable to take up *in vitro* recombinant DNA and to synthesize the proteins encoded by the nucleic acid molecule comprising the nucleotide sequence encoding the ubiquinone-independent DHODH of the present invention. Preferably, the host cell is a cell of humans and non-human organisms, such as primates (e.g. guerilla, macaque, marmoset), livestock animals (e.g. sheep, cow, horse, donkey, pig), companion animals (e.g. dog, cat), laboratory test animals (e.g. mouse, rabbit, rat, guinea pig, hamster), and of any other organism whose cell can be used as a host cell.

Thus, the present invention relates to a ubiquinone-independent DHODH, and/or to a nucleic acid molecule comprising a nucleotide sequence encoding the ubiquinone-independent DHODH, and/or to a recombinant expression vector comprising the nucleic acid molecule comprising the nucleotide sequence encoding the ubiquinone-independent DHODH for use as medicament. More specifically, the present invention relates to a ubiquinone-independent DHODH, and/or to a nucleic acid molecule comprising a nucleotide sequence encoding the ubiquinone-independent DHODH, and/or to a recombinant expression vector comprising the nucleic acid molecule comprising the nucleotide sequence encoding the ubiquinone-independent DHODH for use in the treatment of mitochondrial diseases. Even more specifically, the present invention relates to the ubiquinone-independent DHODH EC 1.3.98.1, and/or to a nucleic acid molecule comprising a nucleotide sequence encoding the ubiquinone-independent yDHODH EC 1.3.98.1, i.e. the *URA1* gene, and/or to a recombinant expression vector comprising the nucleic acid molecule comprising the nucleotide sequence encoding the ubiquinone-independent yDHODH EC 1.3.98.1 for use as medicament. Even more specifically, the present invention relates to the ubiquinone-independent yDHODH EC 1.3.98.1, and/or to a nucleic acid molecule comprising a nucleotide sequence encoding the ubiquinone-independent yDHODH EC 1.3.98.1, i.e. the *URA1* gene, and/or to a recombinant expression vector comprising the nucleic acid molecule comprising the nucleotide sequence encoding the ubiquinone-independent yDHODH EC 1.3.98.1 for use in the treatment of mitochondrial diseases.

According to the present invention, the term "mitochondrial diseases" refers to diseases, which are associated with a defective functional link between the pyrimidine *de novo* biosynthesis and the mitochondrial OXPHOS. Such diseases are caused by alterations or rather mutations which result in a defective or inoperative DHODH catalyzing the fourth step in the pyrimidine *de novo* biosynthesis so that the term "mitochondrial diseases" also refers to diseases, which are accompanied with pyrimidine auxotrophy. More specifically, the term "mitochondrial diseases" refers to to diseases such as Alzheimer's, Parkinson's, and Huntington disease, Miller syndrome, also known as Genee-Wiedemann syndrome, Wildervanck-Smith syndrome or postaxial acrofacial dystosis (POADS), cancer, type 2 diabetes, cardiovascular disease, and osteoarthritis.

The mitochondrial diseases of the present invention occur in a vertebrate or subject to be treated, wherein the term "vertebrate" or "subject" as used herein refers to humans and non-human organisms, such as primates (e.g. guerilla, macaque, marmoset), livestock animals (e.g. sheep, cow, horse, donkey, pig), companion animals (e.g. dog, cat), laboratory test animals (e.g. mouse, rabbit, rat, guinea pig, hamster), and any other organism who can benefit from the medicament of the present invention. There is no limitation on the type of animal that could benefit from the presently described medicament. A vertebrate or subject regardless of whether it is a human or non-human organism may be also referred to a patient, individual, animal, host, or recipient.

The "dosage" of the ubiquinone-independent yDHODH, or rather of the nucleic acid molecule comprising the nucleotide sequence encoding the ubiquinone-independent yDHODH, or rather of the recombinant expression vector comprising the nucleic acid molecule comprising the nucleotide sequence encoding the ubiquinone-independent yDHODH is determined by the physician on the basis of the patient-specific parameters, such as age, weight, sex, severity of the mitochondrial disease. Thus, the therapeutically effective dosage of the ubiquinone-independent yDHODH will vary somewhat from patient to patient and will depend upon the condition of the patient.

In one embodiment of the present invention, the ubiquinone-independent DHODH, or rather the nucleic acid molecule comprising the nucleotide sequence encoding the ubiquinone-independent DHODH, or rather the recombinant expression vector comprising the nucleic acid molecule comprising the nucleotide sequence encoding the ubiquinone-independent DHODH is administered in a dosage of between about 100 milligrams (mg) and 10 grams (g) per day. In another embodiment, the ubiquinone-independent DHODH, or rather the nucleic acid molecule comprising the nucleotide sequence encoding the ubiquinone-independent DHODH, or rather the recombinant expression vector comprising the nucleic acid molecule comprising the nucleotide sequence encoding the ubiquinone-independent DHODH is about 500 mg to 8 g, 750 mg to 7 g, 1 to 6.5 g, 1.5 to 6 g, 2 to 5 g, or 2.5 to 3.5 g per day.

The ubiquinone-independent DHODH, or rather the nucleic acid molecule comprising the nucleotide sequence encoding the ubiquinone-independent DHODH, or rather the recombinant expression vector comprising the nucleic acid molecule comprising the nucleotide sequence encoding the ubiquinone-independent DHODH may be prepared and administered in any dosage form suitable for administration of the subject's body. It is formulated to be compatible with its intended route of administration. Examples of routes of administration include parenteral (e.g. intravenous, intradermal, subcutaneous), oral, by inhalation, systemic (e.g. topical, transmucosal), and rectal administration.

For administration, the above described reagents are preferably combined with suitable pharmaceutical carriers. Examples of pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the vertebrate or subject at a suitable dose.

The present invention also provides an *in vitro* method for diagnosing a mitochondrial disease which is associated with a defective functional link between the pyrimidine *de novo* biosynthesis and the mitochondrial OXPHOS, wherein the method comprises contacting a target sample of a vertebrate or subject suspected to be infected with a mitochondrial disease of the present invention. Transfected cells with a defect in mitochondrial OXPHIS will lose pyrimidine auxotrophy and become prototroph. In this context, patient cells can be easily distingushed by vector complementation so that this technology can be utilized in diagnosis.

The present invention further relates to a non-human transgenic animal.

Techniques for producing transgenic animals (knock-in or knock-out) are well known in the art. A useful general textbook on this subject is Houdebine, Transgenic animals - Generation and Use (Harwood Academic, 1997) - an extensive review if the techniques used to generate transgenic animals from fish to mice and cows. Of particular interest in the context of the present invention are transgenic non-human mammals such as cows, pigs, goats, horses, etc., and particularly rodents, e.g. rats, mice, hamsters, etc. Preferably, the transgenic animal is a mouse.

A "knock-out" of a gene means an alteration in the sequence of the gene that results in a decrease of function of the target gene, preferably such that target gene expression is undetectable or insignificant. A knock-out of an endogenous gene means that function of the gene has been substantially decreased so that expression is not detectable or only present at insignificant levels. "Knock-out" transgenics can be transgenic animals having a heterozygous knock-out of a gene or a homozygous knock-out of a gene. "Knock-outs" also include conditional knock-outs, where alteration of the target gene can occur upon, for example, exposure of the animal to a substance that promotes target gene alteration, introduction of an enzyme that promotes recombination at the target gene site (e.g., Cre in the Cre-lox system), or other method for directing the target gene alteration postnatally.

A "knock-in" of a target gene means an alteration in a host cell genome that results in altered expression (e.g., increased (including ectopic)) of the target gene, e.g., by introduction of an additional copy of the target gene, or by operatively inserting a regulatory sequence that provides for enhanced expression of an endogenous copy of the target gene. "Knock-in" transgenics of interest for the present invention are transgenic animals having a knock-in of a DHODH gene. Such transgenics can be heterozygous knock-in for the DHODH gene or homozygous for the knock-in of the DHODH gene. "Knock-ins" also encompass conditional knock-ins.

Advances in technologies for embryo micromanipulation permit introduction of heterologous DNA into, for example, fertilized mammalian ova. For instance, totipotent or pluripotent stem cells can be transformed by microinjection, calcium phosphate mediated precipitation, liposome fusion, retroviral infection or other means, the transformed cells are then introduced into the embryo, and the embryo then develops into a transgenic animal. In a preferred method, developing embryos are transfected with the desired DNA by electroporation, and transgenic animals produced from the infected embryo. In a further preferred method, however, the appropriate DNAs are coinjected into the pronucleus or cytoplasm of embryos, preferably at the single cell stage, and the embryos allowed to develop into mature transgenic animals. Those techniques are well known. See reviews of standard laboratory procedures for microinjection of heterologous DNAs into mammalian fertilized ova, including Hogan et al., Manipulating the Mouse Embryo, (Cold Spring Harbor Press 1986); Krimpenfort et al., Bio/Technology 9: 844 (1991); Palmiter et al., Cell, 41: 343 (1985); Kraemer et al., Genetic manipulation of the Mammalian Embryo, (Cold Spring Harbor Laboratory Press 1985); Hammer et al., Nature, 315: 680 (1985); Wagner et al., U.S. Pat. No. 5,175,385; Krimpenfort et al., U.S. Pat. No. 5,175,384, the respective contents of which are incorporated herein by reference.

Another method used to produce a transgenic animal involves microinjecting a nucleic acid into pro-nuclear stage eggs by standard methods. Injected eggs are then cultured before transfer into the oviducts of pseudopregnant recipients.

Transgenic animals may also be produced by nuclear transfer technology as described in Schnieke, A.E. et al., 1997, Science, 278: 2130 and Cibelli, J.B. et al., 1998, Science, 280: 1256. Using this method, fibroblasts from the donor animals are stably transfected with a plasmid incorporating the coding sequences for a binding domain or binding partner of interest under the control of regulatory. Stable transfectants are then fused to enucleated oocytes, cultured and transferred into female recipients.

Analysis of animals which may contain transgenetic sequences would typically performed by either PCR or Southern blot analysis following standard methods:
By way of a specific example for the construction of transgenic mammals nucleotide constructs comprising a sequence encoding a binding domain fused to GFP are microinjected using, for example, the technique described in U.S. Pat. No. 4,873,191, into oocytes which are obtained from ovaries freshly removed from the mammal. The oocytes are aspirated from the follicles and allowed to settle before fertilization with thawed frozen sperm capacitated with heparin and prefractionated by Percoll gradient to isolate the motile fraction.

The fertilized oocytes are centrifuged, for example, for eight minutes at 15,000 g to visualize the pronuclei for injection and then cultured from the zygote to morula or blastocyst stage in oviduct tissue-conditioned medium. This medium is prepared by using luminal tissued scraped from oviducts and diluted in culture medium. The zygotes must be placed in the culture medium within two hours following microinjection.

Oestrous is then synchronized in the intended recipient mammals, such as cattle, by administering coprostanol. Oestrous is produced within two days and the embryos are transferred to the recipients 5-7 days after estrous. Successful transfer can be evaluated in the offspring by Southern blot.

Alternatively, the desired constructs can be introduced into embryonic stem cells (ES cells) and the cells cultured to ensure modification by the transgene. The modified cells are then injected into the blastula embryonic stage and the blastulas replaced into pseudopregnant hosts. The resulting offspring are chimeric with respect to the ES and host cells, and nonchimeric strains which exclusively comprise the ES progeny can be obtained using conventional cross-breeding. This technique is described, for example, in WO 91/10741.

Transgenic animals comprise an exogenous nucleic acid sequence present as an extrachromosomal element or stably integrated in all or a portion of its cells, especially in germ cells. It is preferred that a transgenic animal comprises stable changes to the germline sequence. A stable change is generally achieved y introduction of the DNA into the genome of the cell. Vectors for stable integration include plasmids, retroviruses and other animal viruses, YACs, and the like. During the initial construction of the animal, "chimeras" or "chimeric animals" are generated, in which only a subset of cells have the altered genome. Chimeras are primarily used for breeding purposes in order to generate the desired transgenic animal. Animals having a heterozygous alteration are generated by breeding of chimeras. Male and female heterozygotes are typically bred to generate homozygous animals.

In a preferred embodiment, the transgenic non-human mammals of the invention are produced by introducing a DHODH transgene into the germline of the non-human animal. Embryonal stem cell (ES) are the primary type of target cell for introducing of the human C5aR transgene into the non-human animal in order to achieve homologous recombination. ES cells may be obtained from pre-implantation embryos cultured *in vitro* and fused with embryos (Evans, M.J., et al. (1981) Nature 292, 154-156; Bradley, M.O., et al., 9065-9069; and Robertson, et al. (1986) Nature 322, 445-448). Transgenes can be efficiently introduced into the ES cells by DNA transfection or by retrovirus-mediated transduction. Such transformed ES cells can thereafter be combined with blastocysts from a non-human animal. The ES cells thereafter colonize the embryo and contribute to the germ line of the resulting chimeric animal. For review see Jaenisch, R. (1988) Science 240, 1468-1474. The transfected embryonal cells may be incubated *in vitro* for varying amounts of time, or reimplanted into the surrogate host, or both.

Transgenic offspring of the surrogate host may be screened for the presence and/or expression of the transgene by any suitable method. Screening is often accomplished by Southern blot or Northern blot analysis, using a probe that is complementary to at least a portion of the transgene. Western blot analysis using an antibody against the protein encoded by the transgene may be employed as an alternative or additional method for screening for the presence of the transgene product. Typically, in transgenic mice, DNA is prepared from tail tissue and analyzed by Southern analysis or PCR for the transgene. Alternatively, the tissues or cells believed to express the transgene at the highest levels are tested for the presence and expression of the transgene using Southern analysis or PCR, although any tissues are cell types may be used for this analysis. Progeny of the transgenic animals may be obtained by mating the transgenic animal with a suitable partner, or by *in vitro* fertilization of eggs and/or sperm obtained from the transgenic animal.

The following examples show preferred embodiments of the present invention but are not intended to limit the present invention in any aspects.

### Examples

### Material and Methods

Cell lines utilized. In this study, the human cell lines 143B (derived from an osteosarcoma; ECACC 91112502) and 143B.TK- (Thymidine Kinase negative cells display a clear-cut uridine prototrophy) have been utilized. These cell lines were used either in cultures or as parental cells to generate Rho⁰ (ρ⁰) cell lines, which are devoid of endogenous mitochondrial genomes and, thus, exhibiting a uridine auxotrophy.

**Microscope imaging.** Phase contrast and fluorescence imaging were carried out utilizing a Leica DM16000B microscope equipped with a Leica HC PL Fluotar 10x0.30 objective, a Leica DFC350FX camera and Leica filtercubes L5 (EGFP) and N2.1 (DsRed). Images were recorded and processed with the LAS v3.3 software (Leica Microsystems, Wetzlar, Germany) and Adobe Photoshop CS (Adobe Systems, Mountain View, CA). Confocal laser scanning imaging was performed using a Leica TCS SP5 microscope, equipped with a HCX PL APO CS 40.0x1.25 OIL UV objective. All images were acquired in sequential scan mode operating with an argon laser at 488 nm (PicoGreen^{®}) or a DPSS laser at 561 nm (MitoTracker^{®}). Confocal images were recorded and processed applying the LAS AF 2.3.6 software (Leica Microsystems and Adobe Photoshop CS (Adobe Systems).

**Generation of colored cell lines.** Cell lines harbouring green or red mitochondria were generated by transfecting 143B, HeLa or derivatives thereof with pEGFP-Mito (based on pECFP-Mito vector of Clontech with ECFP replaced by EGFP) or pDsRed1-Mito vector (Clontech, Mountain View, CA, USA) utilizing Metafectene^{®} (Biontex, Planegg/Martinsried, Germany) according to the protocol provided by the supplier. Transfected cells were selected by complementing culture medium with neomycin sulfate (AppliChem, Darmstadt, Germany).

**Generation of Rho⁰ cell lines.** To generate Rho⁰ cell lines, parental cells (143B, HeLa) were treated with mitogenomix™ delta lipo mtDNA depletion system (RhoZero Technologies, Uettingen, Germany) following the recommendations of the supplier. In short, culture media were supplemented with uridine and pyruvate. Cells were temporarily transfected with the mitogenomix™ delta vector that contained a mitochondrially targeted restriction endonuclease cleaving mtDNA to induce self-destruction by endogenous nucleases. Two days post transfection, single cells were isolated, grown (cloning) and checked for Rho⁰ cell state by PCR and metabolic testing as described elsewhere (Kukat, A. et al., Nucleic Acids Res 36, e44 (2008)).

**FACS analyses.** Cells utilized in FACS analyses (143B.TK⁻.p⁰, 143B.TK⁻.p⁰. rvDHODH-IRES-EGFP) were cultured in normal growth medium (DMEM supplemented with 5% dialyzed FCS, 4.5 g/l glucose, pyruvate, 1% BrdU, 0.5% uridine and glutamine). Prior to FACS, cells were trypsinized and seeded at 0.4*10⁶ cells/dish. 24 hours post seeding (day 0) growth medium was replaced by uridine-free media. Cell samples were taken at different time intervalls of uridine starvation (0h, 3h, 6h, 9h, 12h, 2d and 5d). In short, cells were trypsinized, washed and stored resuspended at 4°C in PBS-EDTA/EtOH solution over night. FACS analyses were carried out in the presence of propidium iodide solution (Sigma-Aldrich) following the recommendation of the supplier.

**Complementation of Rho⁰ cells by expressing URA1 from S. *cerevisiae.*** Genomic DNA extracted from *S*. *cerevisiae* was used as PCR template to amplify the coding sequence of the fumarate depending dehydroorotate dehydrogenase (URA1; EC 1.3.98.1) utilizing the primers SCER-URA1-001-FOR and SCER-URA1-944-REV.

The amplified fragment contained 5' and 3' modified ends so that a site directed cloning via the restriction sites Nhel and Pstl was enabled. Initially, the amplified fragment was cloned in a pCRII-TOPO vector (Life Technologies, Darmstadt, Germany), analyzed by sequencing, prepared (cleaved with *Nhe*I and *Pst*I) and treated with T4 DNA polymerase to yield blunt ends for a further cloning step in the retrovirus vector pLXRN (Clontech, Mountain View, CA). In short, pLXRN plasmid was linearized utilizing *Bam*HI, treated with T4 DNA polymerase to yield blunt ends and finally ligated to the prepared pLXRN vector named pLXRN-DHODH.

SCER-URA1-001-FOR: attcGCTAGC-atgacagccagtttaactaccaagttcttg SCER-URA1-944-REV: aattcCTGCAG-ttaaatgctgttcaacttcccacgg (capital letters: indicating restriction site located in 5'-overhang; first nucleotide downstream of the restriction site represents first or last (1 or 944) nucleotide of the coding sequence. Primers and enzymes utilized in this study were purchased from Fermentas/Thermo).

To simplify identification of viral infected cells afterwards, an optical marker that does not interfere with DHODH activity has been introduced. In this context, the IRES2-EGFP region has been taken from a pIRES2-EGFP vector by *Pst*I / *Not*I cleavage, the fragment has been treated with T4 DNA polymerase to yield blunt ends and has been ligated to the prepared pLXRN-DHODH (cleaved with *Xho*I and treated with T4 DNA polymerase to yield blunt ends). The resulting vector (pLXRN-DHODH-IRES2-EGFP; see Fig. 2) was verified by sequencing and transfected into RetroPack™ PT67 cells (Clontech, Mountain View, CA) to generate an infective supernatant according to supplier's protocol.

Further on, the parental 143B cells and their Rho⁰ derivatives were treated with the retrovirus (rv-pLXRN-DHODH-IRES2-EGFP) according to manufacturer's recommendation to complement uridine metabolism.

### Growth Media:

Without uridine:
- DMEM, 4,5 g/l Glucose, stab. Glutamin
- 5% FCS
- 1 mM Natriumpyruvat
- 100 µg/ml Bromdesoxyuridin

With uridine:
Same as above +50 µg/ml Uridin

### Example 1

### Infection of 143B.TK⁻ cells or 143B.TK⁻.Rho⁰ cells with the recombinant expression vector introducing S. cerevisiae yDHODH

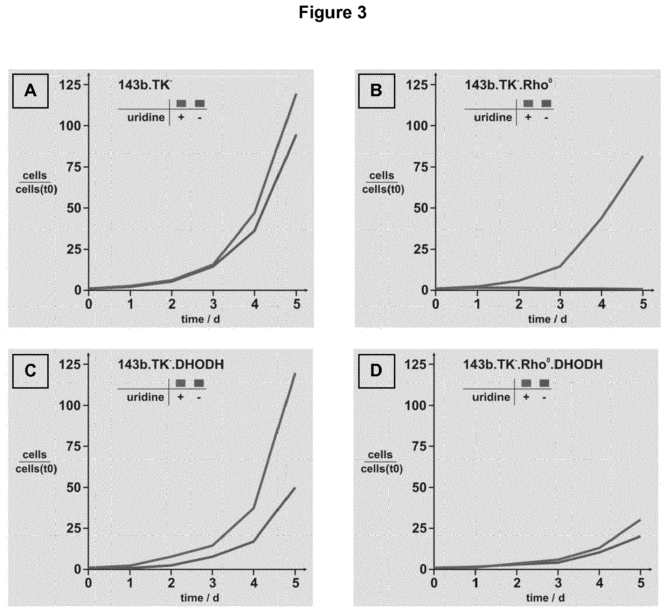

When infecting 143B.TK- cells or 143B.TK⁻.Rho⁰ cells with the recombinant expression vector introducing *S*. *cerevisiae* yDHODH, cells could be grown in the presence or absence of uridine (Fig. 3c and 3d). The expression of yDHODH in 143B.TK- cells slightly decreased the cellular growth rate (Fig. 3c), which could be ascribed to the unphysiological branching of uridine synthesis and/or compartmentalization, due to the simultaneous activity of both the cytoplasmic and mitochondrial enzyme. Furthermore, the growth rate of 143B.TK-.Rho⁰.DHODH cells in the absence of uridine was practically comparable to that in the presence of uridine (Fig. 3d), which demonstrates that the uridine auxotrophy of Rho⁰ cells can be rescued by expressing the ubiquinone-independent yDHODH.

### Example 2

### Culturing of 143B.TK⁻.Rho⁰ cells and 143B.TK-.Rho⁰.DHODH cells in the absence of uridine

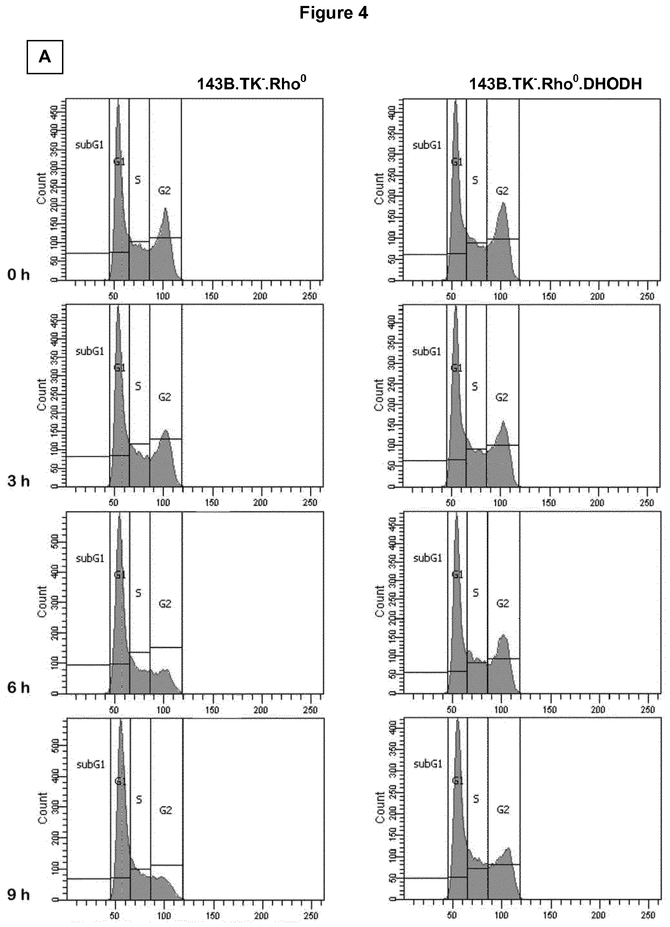

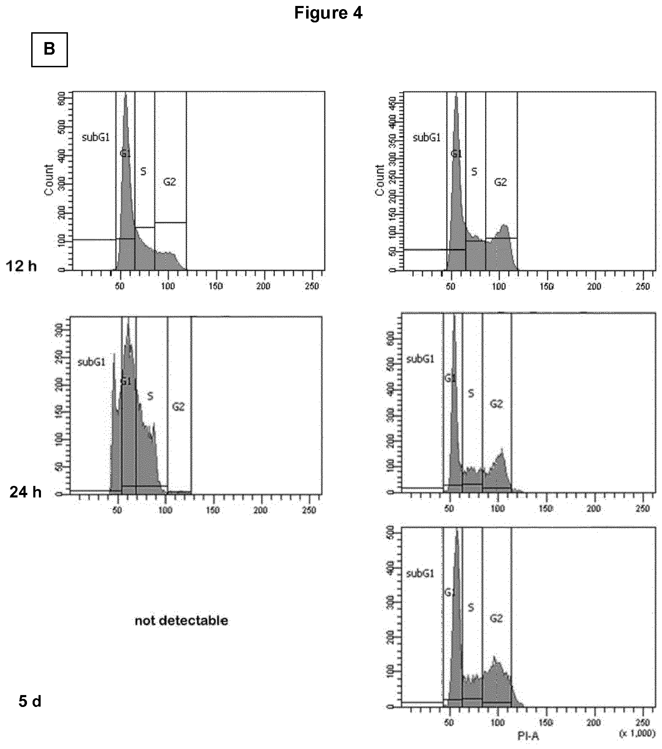

It turned out that the cell cycle of 143B.TK⁻.Rho⁰ cells stalls within 12 to 72 hours of uridine withdrawal, as shown by the decrease in the count of G2 and S phase cells and the relative increase in the count of G1 phase cells (see Fig. 4). However, cell growth could be reinitiated by a uridine pulse that unlocked cell cycle arrest, thus, allowing cells to proceed mitosis. If 143B.TK⁻.Rho⁰ cells were kept for more than 48-80 hours without uridine supply, cell death was irreversibly triggered as demonstrated by sharp appearance, at this time, of the subG1 phase cells signal. However, the expression of yDHODH in 143B.TK-.Rho⁰.DHODH cells abolished the cell cycle arrest and the consequent cell death.

### Example 3

### Release of 143B.TK⁻.Rho⁰ cells from cell cycle arrest by uridine complementation

143B.TK⁻.Rho⁰ cells have been cultured for approximately 12 hours without uridine supplementation, 10 hours post uridine supplementation. The white arrowheads in Fig. 5 indicate cells in metaphase/anaphase transition, indicating that cell cycle arrest proceeded from G1 to mitosis upon uridine supplementation (red color: mito-dsRED illuminating mitochondrial network; green color: PicoGreen staining the nuclear DNA). Thus, the observed cell cycle arrest was released by supplementing the medium with uridine.

## Claims

1. Ubiquinone-independent cytoplasmic dehydroorotate dehydrogenase (DHODH) for use as medicament.

2. Ubiquinone-independent DHODH according to claim 1, wherein the ubiquinone-indpendent DHODH originates from *Saccharomyces cerevisiae.*

3. Ubiquinone-independent DHODH according to claim 2, wherein the ubiquinone-indpendent DHODH is the Class 1 DHODH, EC 1.3.98.1.

4. Ubiquinone-independent DHODH according to any of claims 1 to 3 for use in the treatment of a mitochondrial disease.

5. Ubiquinone-independent DHODH according to any of claims 1 to 3 for use according to claim 4, wherein the mitochondrial disease is associated with a defective mitochondrial link between the pyrimidine *de novo* biosynthesis and the mitochondrial oxidative phosphorylation system (OXPHOS).

6. Ubiquinone-independent DHODH according to any of claims 1 to 3 for use according to claim 4 or 5, wherein the mitochondrial disease is selected from the group consisting of Alzheimer's, Parkinson's, and Huntington disease, Miller syndrome, cancer, type 2 diabetes, cardiovascular disease, and osteoarthritis.

7. Nucleic acid molecule comprising a nucleotide sequence encoding the DHODH according to any of claims 1 to 6 for use according to any of claim 1 to 6.

8. Recombinant expression vector comprising the nucleic acid molecule according to claim 7 for use according to any of claims 1 to 6.

9. Recombinant host cell comprising the recombinant expression vector according to claim 8 for use according to any of claims 1 to 6.


SEQUENCE LISTING

<110>  Universität Leipzig

<120>  Ubiquinone-independent cytoplasmic dihydroorotate dehydrogenase
       for use as medicament

<130>  U 1126EP

<140>  EP17165728.1
<141>  2017-04-10

<160>  3

<170>  PatentIn version 3.5

<210>  1
<211>  40
<212>  DNA
<213>  artificial sequence

<220>
<223>  primer forward

<400>  1
attcgctagc atgacagcca gtttaactac caagttcttg                          40


<210>  2
<211>  36
<212>  DNA
<213>  artificial sequence

<220>
<223>  primer reverse

<400>  2
aattcctgca gttaaatgct gttcaacttc ccacgg                              36


<210>  3
<211>  945
<212>  DNA
<213>  Saccharomyces cerevisiae

<400>  3
atgacagcca gtttaactac caagttcttg aacaatacct atgaaaaccc atttatgaat      60

gcatccggtg ttcattgcat gactacacaa gaattagatg aattagcaaa ctctaaagct     120

ggcgcattca ttacaaagag tgctacaacc ttagaaagag aaggtaaccc tgaaccacgt     180

tacatttctg tccctctagg cagtatcaac tccatgggtt taccaaacga aggtatcgac     240

tactatttgt cctatgtatt aaaccgtcaa aagaattatc ctgatgcacc tgctattttc     300

ttctcagttg ctggtatgag cattgatgaa aatttaaatt tgttgaggaa aatccaagat     360

agcgaattca acggtattac cgagttaaac ttgtcttgtc ctaatgtgcc tgggaaacca     420

caagttgctt atgactttga cttgacaaag gaaaccttgg aaaaggtttt tgcctttttc     480

aaaaaacctc ttggtgtcaa gttgcctcct tattttgatt ttgcccattt tgatatcatg     540

gcaaaaatat tgaacgagtt cccattagct tatgtcaact ctatcaatag tataggaaat 600

ggtcttttca ttgatgtgga gaaggagagt gtagtagtga agccaaagaa tggtttcggg 660

ggtattggag gtgaatatgt taagccaacc gcgctcgcca atgttcgtgc attttacact 720

cgtttgagac ctgaaatcaa agttatcggt acaggtggaa ttaagtccgg taaggatgca 780

tttgaacatc ttctatgtgg tgcctctatg ctacagattg gtacagaatt acaaaaagag 840

ggcgtcaaga tttttgaacg tatcgaaaaa gaattaaaag acataatgga agctaagggt 900

tatacatcca tagatcagtt ccgtgggaag ttgaacagca tttaa 945